# EUROPEAN PATENT SPECIFICATION

(11) **EP 1 676 546 B1**
(45) Date of publication and mention of the grant of the patent: **30.11.2011**
(21) Application number: 05257969.5
(22) Date of filing: 22.12.2005
(51) Int. Cl.: A61F 2/01

(54) **Distal protection apparatus with improved wall apposition**
Distaler Schutzfilter mit verbesserter Apposition zu den Wänden
Filtre de protection distal avec apposition améliorée

(30) Priority: 30.12.2004 US 26377
(43) Date of publication of application: 05.07.2006
(73) Proprietor: Cordis Corporation, Miami Lakes, FL 33014 (US)
(72) Inventor: Arguello, Edward, Miramar FL 33027 (US); Wang, Huisun, Miramar FL 33027 (US); Widenhouse, Christopher William, Cincinnati OH 45249 (US); Wijeratne, Lalith Hiran, Cooper City FL 33328 (US)
(74) Representative: Gover, Richard Paul

(56) References cited:
- EP-A- 1 386 624
- WO-A-03/026532
- US-A1- 2003 065 356
- US-A1- 2003 130 684
- US-A1- 2003 150 821
- US-A1- 2004 220 608
- US-B1- 6 348 062

## Description

The present invention relates to intravascular devices used to assist in medical treatment and procedures. More specifically, the present invention relates to a blood filtering system for preventing embolic material from migrating through a blood vessel during an intravascular procedure.

Atherosclerosis is a complex disease, being primarily a result of buildup in the arteries that may start as early as childhood and progress as one ages. Progression may be rapid in some people. Blood vessels may become completely occluded or more often narrowed and/or stenotic in a number of ways. A stenosis may be formed by an atheroma which is typically a harder, calcified substance which forms on the inside walls of the blood vessel. The stenosis may also be formed by a buildup of thrombus material, which may restrict blood flow through the vessel. In general, atherosclerosis is the result of any combination of fat substances, cholesterol, waste products, calcium as well as other substances being deposited on the inside lining of the artery. This buildup is often called plaque. Atherosclerosis can often lead to coronary heart disease, a major health concern today for both males and females in the United States as well as abroad.

Atherosclerosis may also lead to strokes, and other disorders because of the occurrence of blood clots, which may form in the narrowed arteries. Although plaques can grow large enough to significantly reduce the blood flow through an artery, most of the damage may occur when these plaques become fragile and rupture. These are often referred to as vulnerable plaques. When vulnerable plaques rupture they typically cause blood clots to form that may then subsequently block blood flow or break off and travel through the blood vessel to another part of the body. If either situation happens, the result may be a blocked blood vessel that supports and nourishes the heart, which may cause a heart attack. If a blood vessel that delivers blood to the brain is blocked, it may cause a stroke. If the blood supply to the legs is compromised, it may result in limb ischemia and in difficulty with walking and/or leg pain referred to as claudication, and may eventually cause gangrene.

The narrowing of an artery, also known as a stenotic lesion in the vasculature, has motivated medical professionals to develop a number of intravascular procedures which have evolved over time to treat this condition, percutaneous balloon angioplasty being the most common. Percutaneous balloon angioplasty is a procedure wherein a balloon catheter is inserted within the vasculature, and the balloon is expanded at the location of the lesion essentially compressing the stenotic buildup against the inside of the vessel wall. More recently this procedure has been augmented by the deployment of a stent or stents, at the location of the lesion subsequent to, or concurrently with the angioplasty. The stent acts as an internal scaffold within the vessel, retaining an open lumen and preventing further re-narrowing of the vessel. Generally, stents are primarily of two types, balloon expanding and self-expanding. As the terms indicate, balloon-expanding stents are deployed/expanded *in-vivo* with the assistance of a balloon, while self-expanding stents may utilize shape-memory materials such as nitinol. The use of such alloys as nitinol (Nickel-Titanium alloy), having shape memory characteristics, suitable biocompatibility, and designed to be inserted into one's vasculature, is known in the art. These so-called shape memory alloys have the ability to return to their original shape when exposed to the correct temperature conditions. The shape memory characteristics of these alloys, allow the devices to be deformed to facilitate their insertion into a body lumen or cavity. Upon being exposed to higher temperatures within the body results in the device returning to its original programmed shape. Thus one can employ the shape memory property to expand the device to its original shape after being delivered through the vasculature in a reduced profile or compressed state. Nitinol can also have super-elastic characteristics, which allow the device fabricated from such a material as nitinol, to be deformed and restrained in the deformed condition in order to facilitate the insertion into a patient's vasculature. This deformation causes a phase transformation of the material. Once the device with super-elastic characteristics is delivered, the restraint on the super-elastic material can be removed thus reducing the stress and allowing the previously compressed super-elastic member to return to its original pre-programmed and un-deformed shape, which results in a transformation back to the original phase.

While widespread intravascular procedures, particularly angioplasty procedures have been extremely successful; the procedure itself may result in development of an embolus. For example, during stent deployment and positioning, abrasion of the vessel wall may dislodge material resulting in an embolus. An embolus circulating within the blood vessels may lead to occlusion of a vessel and/or formation of clots within the vasculature and/or body organs. Although the occurrence of this can be minimized with careful and proper technique, when such an event does happen it may have serious consequences to the patient. By adequately capturing and/or filtering the material traveling in the blood responsible for causing such an event one can avoid the serious consequences that may result without such safeguards.

Multiple approaches to address capturing and/or filtering of the embolic debris/material from blood have been attempted. These include baskets, nets, suction, and even chemical modification of the debris (see US-5053008, which utilizes and requires an additional conduit to transport lysing agents to the trapped embolus). Use of vascular filters in the Vena Cava for capturing emboli has been disclosed (see US-4727873 and US-4688553). The designs of the Vena Cava filters continue to improve addressing such issues as fit and preventing migration (see US-6443972). Distal Protection devices using filtering baskets although similar to Vena Cava filters in function are typically temporarily positioned within the lumen, whereas Vena Cava filters are typically implanted within the vessel, most often the inferior Vena Cava, as the name implies. The use of a filtering basket positioned downstream from the procedure to capture the debris/material during an intravascular procedure is one such method. Because these basket type devices must be introduced into the vasculature and travel within the vasculature to be ultimately positioned to a location somewhat distal or downstream to the region of interest, most, if not all, incorporate and utilize concepts and features that make the essential delivery through the vessel less traumatic (see US-6391044). This can be accomplished by using a reduced size or compressed version of the apparatus. This allows one to deploy the apparatus to its normal working size when the apparatus is at the location of interest within the vessel.

The majority of these basket-type devices employ "expandable filters," meaning they may be fabricated from shape-memory materials which have the property that when exposed to the relatively elevated temperature within the body, they return to their initial programmed size. Alternately, on can rely on the superelastic property by removing the restraint on the geometry. These devices are generally placed distal, or downstream of the stenosis in order to capture any fragments, debris, and/or embolic material, that may be dislodged or occur as a result of the presence and use of the device during an intravascular procedure. The downstream placement of the device takes advantage of the blood flow within the vasculature, which will transport the undesirable material with it. The filtering membrane of the device is typically designed so as to allow blood flow through the membrane while limiting passage of the larger sized fragments and debris such as micro and macro emboli. These fragments, debris, and/or embolic material could potentially be carried beyond the device location with the blood flow downstream if not for such a filtering device as described herein. While this method performs fairly well capturing a substantial portion of the items intended to be captured; many of these designs are optimized for circular vessels. While outer vessel shapes are circular or slightly elliptical, the internal geometry of a vessel may often be non-circular as in an oval or elliptical shape or may even take the form of other non-circular geometries. Specifically, when calcification is present within the vessel, the internal vessel geometry is often irregular. Furthermore, vessel cross-sectional shape may vary with the type and location of vessel and vary across patients as well. Moreover, due to the circulation of blood through the vessel and resulting forces, a dynamic situation exists, which may produce additional geometry changes to the normal vessel shape. Thus expandable filters, which are generally designed for circular vessels, may result in a lack of apposition against the vessel wall over at least some portion of the internal circumference of the vessel wall when one takes into account the additional factors described above. Such a gap or leak path may occur when the resulting geometry of the expanded device is circular while the inner luminal cross-sectional shape of the vessel is more often non-circular. Such resulting gaps, between the device and inner luminal surface, may allow the emboli that the device is designed to capture, adequate room to pass through such a gap between the inner wall of the vessel and the outer confines of the device. When this occurs, the primary purpose of the device, which in this case is to capture fragments, debris and/or embolic material, is defeated, because the unfiltered flow path will allow for passage of the emboli. Even when the vessel itself is circular, conformance of a circular filter to the internal lumen of the vessel may not be optimal if "in-folding" is present. "In-folding" is the situation when the unsupported membrane of the system folds in at positions located between the strut locations where the membrane is supported by the struts. This situation can produce gaps between the inner vessel wall and the membrane even in the idealized circular vessel at locations between adjacent struts. In-folding may also occur when an oversized device (one that is sized larger then the vessel it will be placed in) is utilized in order to ensure adequate vessel coverage. In this situation, when the struts of the oversized device make contact with the vessel wall, the device ceases to expand. As a result, this limited expansion is short of its fully expanded programmed size and as such the membrane is not fully taught. Thus the remaining slack present in the membrane may lead to in-folding and thus allow for an unfiltered flow path. Moreover, even in the absence of in-folding, utilization of a circular-type device in a truly circular vessel may not adequately conform to the internal lumen upon vessel loading and/or deformation because the resulting device deformation may not adequately match the deformation of the vessel. This dissimilar deformation may thereby result in gaps or leak paths between the inner vessel wall and the device upon loading and/or deformation of the vessel.

WO-03/026532 discloses medical devices comprising nanomaterials. In particular, a device for insertion into a bodily lumen is disclosed including longitudinal extending support ribs coupled at proximal ends to a collar. An orifice is formed from a pleated ring attached to the distal ends of the support ribs. The pleated ring supports a filter membrane.

US-2004/0220608 relates to a radiopaque embolic protection frame. It discloses an expandable frame comprising a pair of half frames which expand a filter element into a deployed position.

US-2003/0130684 relates to a support frame for an embolic protection device. The device includes round frame wires that comprise support hoops for a filter body.

US-6348062 relates to a vascular device having one or more articulation regions. A construction is disclosed comprising a sac formed from a thin flexible material and a support hoop that folds in half and collapses to fit within a small diameter delivery sheath.

Accordingly, there is a need for a distal protection device with improved filtering and vessel apposition that can allow for capturing of embolic debris regardless of vessel size or shape or various loading regimes encountered by the vessel.

The distal protection device with improved apposition in accordance with the present invention overcomes the disadvantages and shortcomings of currently available devices and satisfies the unmet needs of maximizing capture of embolic debris by improved vessel apposition in vessels of varying size and shape in various loading and no-load regimes.

The present invention relates to an apparatus for intravascular filtering, capable of capturing emboli in blood flowing within the vasculature and a method of using the device. The filtering device comprises an expandable basket and is configured to deploy radially outward which may be relative to a centrally located guide wire. Expansion of the filtering device with improved vessel conformance is accomplished in a fashion resulting in improved filtering of blood in both circular and non-circular vessels and for vessels both large and small as well as when the vessel itself encounters various internal and/or external loading regimes.. Furthermore the incorporation or application of biological and/or pharmaceutical agents can provide additional benefits when used in combination with the present invention.

According to a first aspect of the present invention there is provided a vascular filtering device comprising: a compliant substantially enclosed filter membrane having a proximal opening; a closure ring having a concentric though hole adapted to slidably engage with a guide wire; and two or more supporting struts spaced apart from one another having proximal terminal ends, distal terminal ends, and substantially circumferential intermediate portions between the terminal ends, wherein the terminal ends are operatively attached to said closure ring; characterized in that the circumferential intermediate portions of said supporting struts are operatively attached to the proximal opening of said filter membrane such that each supporting strut can expand away from a guide wire independently of the or each other supporting strut such that radial expansion of the filter membrane is non-uniform.

According to a second aspect of the present invention there is provided a method for fabricating a filtering device comprising the steps of: providing elongated wire; forming said elongated wire into strut members; and attaching said strut members to a closure ring having a concentric through hole adapted to slidably engage a guide wire, wherein each strut member has a proximal terminal end, a distal terminal end and a substantially circumferential intermediate portion between the terminal ends, wherein the terminal ends are operatively attached to said closure ring; characterized in that the circumferential intermediate portions of said strut members are operatively attached to a proximal opening of a compliant substantially enclosed filter membrane such that each strut member can expand away from a guide wire independently of the or each other strut member such that radial expansion of the filter membrane is non-uniform.

In accordance with one exemplary embodiment of the present invention, the filtering basket and supporting struts work together to achieve the stated objective of improved conformance and apposition to the internal vessel wall. In this exemplary embodiment of the present invention where the filtering basket and struts work together, expansion of the filtering basket is decoupled from the struts, and as such, expansion of the filter basket can be independent of the expansion of the supporting struts that are operatively connected to the filtering basket. Operatively connected in this instance in accordance with an exemplary embodiments of the present invention equates to the struts connected to the membrane such that radial strut movement controls the radial expansion of the membrane but allows for independent longitudinal or axial movement of strut relative to the membrane. For example, in accordance with the present invention one can operatively attach the strut to the membrane by allowing the strut to slide within loops fixed to the membrane thereby allowing the radial expansion of the membrane to occur independent of the axial translation of the individual struts. In this exemplary embodiment, the independent expansion of each of the supporting struts can independently act on the filtering basket enabling the basket to expand as well. This expansion of the filtering basket may be non-uniform. As a result, improved vessel wall apposition is achieved by the independent radial expansion of each of the struts acting upon the filtering portion of the device. This improved conformance to the inside surface of an vessel wall results in improved filtering capacity by capturing emboli and/or other material which may otherwise be allowed to circumvent a device which does not conform to the vessel wall as closely. This provides a significant patient benefit.

In another exemplary embodiment of the present invention, one can improve conformance with the internal lumen of the vessel while further reducing the folded and/or compressed profile of the system by utilizing a series of independent struts optimized for improved apposition in both circular and non-circular vessels. This can be achieved by utilizing independent strut loops that occupy only the proximal portion of the filter thereby further reducing the compressed profile of the distal portion of the filter due to the absence of struts from the distal portion of the filter basket. Each independent strut loop can expand in a similar or dissimilar fashion relative to the additional strut loops, which allows for the improved conformance of the membrane to the vessel wall. In accordance with the present invention, examples of strut loops can include "U" and "V" type loops. The looping of the strut is achieved by and best described as a substantially axial portion of the strut, which is then directed circumferentially for a portion of the circumference, the path of the strut then following a second substantially axial portion returning to the approximate starting position of the strut and completing the loop. In "U" type loops, the circumferential portion of the loop in combination with the two axial portions can result in a shape similar to the letter "U", alternately in "V" type configurations, the distal axial portions of the strut may form a shape similar to the letter "V". The "V" type configuration, which facilitates easier and more efficient compression and/or folding of the device, due to the distal axial portions which can be aligned when compressed, results in reduced profile dimensions which is extremely important given the delivery considerations through the vasculature to the region of interest. The "V" type configuration may also be cut from smaller profile tubing. It is important to note that any suitable configuration can be utilized. The circumferential portion of the strut, which is operatively attached to the membrane, allows the extent of in-folding to be minimized while achieving improved vessel conformance, because the circumferential portion of the strut ensures conformance with the vessel wall by providing support to the membrane which is adjacent to the vessel wall. Since the struts in combination with the compressed or folded filtering membrane is the primary contributor to profile in the distal region of the device, limiting the strut loop to the proximal portion of the device minimizes the overall profile of the device due to the absence of struts in the distal portion.

The incorporation or application of biologically active or pharmaceutically active compounds with the present invention is a further object of this invention and is an improvement to methods and/or devices which require the use of a conduit to deliver the agent to the desired location. Compounds such as those identified below may be applied as coatings on these devices and may be used to deliver therapeutic and pharmaceutical agents which may include: anti-proliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP) 11_{b}/111ₐ inhibitors and vitronectin receptor antagonists; anti-proliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes - dacarbazinine (DTIC); anti-proliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (i.e. estrogen); anti-coagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); anti-inflammatory: such as adrenocortical steroids (cortisol, cortisone, fludrocortisone, prednisone, prednisolone, 6α-methylprednisolone, triamcinolone, betamethasone, and dexamethasone), non-steroidal agents (salicylic acid derivatives i.e. aspirin; para-aminophenol derivatives i.e. acetaminophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF); angiotensin receptor blockers; nitric oxide donors; antisense oligionucleotides and combinations thereof; cell cycle inhibitors, mTOR inhibitors, and growth factor receptor signal transduction kinase inhibitors; retenoids; cyclin/CDK inhibitors; HMG co-enzyme reductase inhibitors (statins); and protease inhibitors.

The use of compounds in conjunction with the present invention can provide distinct clinical advantages over existing therapies and/or devices. More specifically, compounds that are capable of causing lysis or degradation of the embolic debris can be incorporated into the filtering portion of the present invention. A factor to consider in the selection of such a compound is the origin of the debris be it thrombus, plaque, atheroma, or any other form representing an embolus. As the mesh and or pore size of the filtering aspect of the present invention decreases, more embolic material may become trapped in the filtering mechanism of the present invention, thereby increasing the load on the filtering portion. While small emboli (typically smaller than 100 µm (100 microns)) are not a major concern because of the body's natural ability to enzymatically degrade, digest or lyse the emboli, the embolic load on the filter itself can be overloaded and result in formation of a thrombus if the blood flow is significantly slowed to the point which allows for a thrombus formation. In this situation the incorporation or application of compounds, which can degrade trapped emboli, can be beneficial. Some exemplary suitable compounds may include: Tissue Plasminogen(TPA); Streptokinase(SK); Reteplase; Tenecteplase; Urokinase; Lanoteplase; Staphylokinase; and/or Nadroparin(anti-factor Xa). In addition, the filtering portion of the present invention may incorporate an antithrombotic and/or antithrombogenic agent to prevent the formation of a thrombus. Some exemplary compounds may include: Heparin; Fragmin (dalteparin, low MW Heparin); a monoclonal antibody such as ReoPro™ (abciximab, antiplatelet antibodies) Acenocoumarol; Anisindione; Dicumarol; Warfarin; Enoxaparin (Lovenox); Anagrelide (Agrylin); Indomethacin (Indocin); Dipyridamole; Clopidogrel; Aggrenox; and/or Coumadin. Furthermore, an affinity-binding compound may also be incorporated with the filtering aspect of the present invention by itself or in combination with other compounds. Affinity-binding compounds can promote the binding and/or adhesion of embolic material thus facilitating entrapment of embolic material and subsequent removal from the blood stream. Whether incorporated into the strut or membrane by methods such as chemical surface treatments, bombardment, placement into reservoirs, or in the case of polymeric struts and membranes, blended with the material itself, or by application of a coating to the struts and/or membranes with a compound, any identified compound or combination of identified compounds may be used. Furthermore any number of compounds may suggest themselves to one who is skilled in the art and may be utilized in connection with the present invention alone or in combination with other compounds.

The foregoing exemplary embodiments of the present invention each provide a reliable, easy to manufacture, and simple to use device that significantly improves wall apposition of the outer confines of the device to the internal surface of the vessel wall regardless of vessel size or shape or loading regime encountered. Furthermore, a relatively reduced profile of the delivered device is achievable in accordance with the present invention. Moreover, any combination of the items identified that are capable of expansion and provide for the ability to independently conform to both circular and non-circular geometries upon expansion may be utilized. As noted above, the incorporation of biological and/or pharmaceutically active agents with the present invention can be utilized for the additional purposes of preventing thrombus formation, promotion of binding, and degradation of thrombus, all of which provide a patient benefit.

Embodiments of the invention will now be described by way of example with reference to the accompanying drawings, in which:
Figures 1a & 1b are planar views showing the disparate cross-sectional coverage area of an existing prior art filtering device in both a circular vessel and a non-circular vessel and the resulting gap in coverage due to non-conformance of the device when positioned in non-circular vessels;
Figures 2a & 2b are planar views showing the disparate cross-sectional coverage area by a filter with improved vessel conformance in accordance with the present invention in both a circular and non-circular vessel. The schematic in figure 2b shows the minimization of gaps particularly in a non-circular vessel that results in contrast to the result achieved with prior art devices shown schematically in figure 1b;
Figure 3a is a three-dimensional perspective view incorporating four strut loops of the "U" type configuration, operatively attached to a filtering membrane in accordance with the present invention;
Figure 3b is a three-dimensional perspective view incorporating three strut loops of the "U" type configuration operatively attached to a filtering membrane in accordance with the present invention;
Figure 4a is a partial side view of the four-loop embodiment of the "U" type configuration shown in figure 10a in accordance with the present invention;
Figure 4b is a partial side view of the four-loop embodiment of the "U" type configuration shown in figure 10a with additional filtering membrane support in accordance with the present invention;
Figure 4c is a partial side view of the four-loop embodiment of the "U" type configuration shown in figure 10a showing a scalloped membrane allowing for additional reductions in profile in accordance with the present invention;
Figure 4d is a partial side view of the four-loop embodiment of the "V" type configuration shown in figure 10a showing a scalloped membrane allowing for additional reductions in profile in accordance with the present invention; and
Figure 4e is a partial side view of the four-loop embodiment of the "U" type configuration shown in figure 10a showing both additional filtering membrane support and a scalloped membrane in accordance with the present invention.

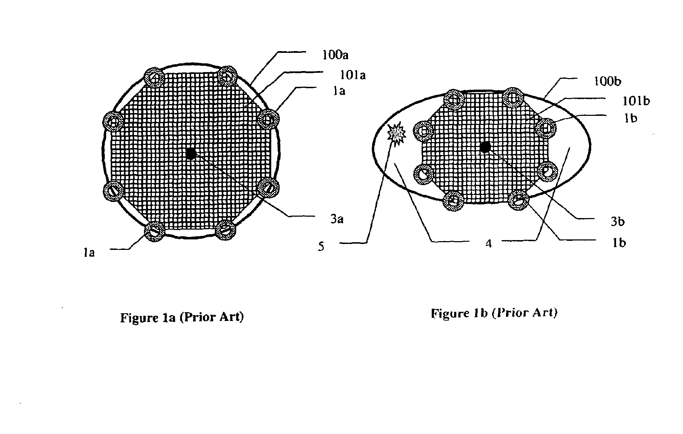

Figures 1a & 1b show a cross-sectional view for both a circular (100a) and a non-circular (100b) blood vessel. Superimposed within these vessels in figures 1a & 1b are schematics showing the approximated or relative cross-sectional coverage area (101a) and (101b) for typical existing prior art devices in both circular (100a) and non-circular (100b) idealized vessels. In this case the vessel wall (100a) & (100b) constrains the extent of the expansion of devices located within the circumference of the vessel. With existing devices that uniformly expand, this expansion is such that strut point locations represented by (1a) & (1b) in the circular and non-circular vessels respectively, are uniformly equidistant from the central axis (3a & 3b). As such, when strut point locations (1b) come into contact with the vessel wall (100b), at the moment one or more struts make contact, all subsequent expansion is halted. As shown, given that all strut point locations (1b) expand in unison and uniformly, the right two and left two strut point locations (1b) never make contact with the internal vessel wall surface (100b) because the top two and bottom two strut point locations (1b) make prior contact with the vessel wall (100b) and thus the entire device is prevented from further expansion. This results in a gap of coverage area (4) that may allow embolic material (5) to flow past the filtering device. The gap in coverage area is not only present in non-circular vessels, but depending on the extent of non-circularity of the vessel may result in further increasing the gap present and may also occur or increase due to various vessel loading situations.

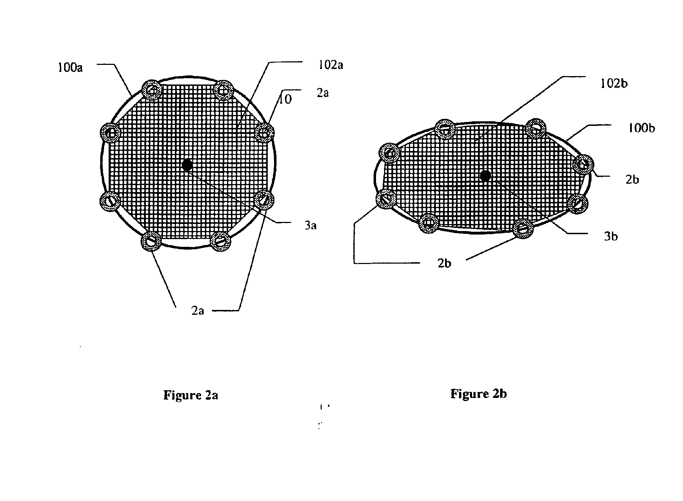

This is in contrast with the result achieved with an improved vessel conformance device in accordance with the present invention. A similar set of schematics represented in Figures 2a & 2b show the cross-sectional coverage area and obtainable results in accordance with the present invention in both circular (100a) and non-circular (100b) vessels. Figures 2a & 2b show cross-sectional views for both a circular (100a) and a non-circular (100b) blood vessel. Superimposed within this vessel is a schematic showing the cross-sectional coverage area (102a) and (102b) for an improved vessel conformance devices in accordance with the present invention in both circular (100a) and non-circular (100b) vessels. In this case the vessel wall (100a) & (100b) constrains the extent of the expansion of devices located within the circumference of the vessel as before. However because the strut point locations (2a & 2b) are decoupled from the filtering portion these strut points can continue to expand independently of each other and the filtering basket, even when one strut point location makes contact with the vessel wall. Thus the outward expansion of the other strut points (2b) are not inhibited and thus can continue to expand until each independently makes contact with the vessel wall regardless of the nature of the cross-sectional shape of the vessel. This results in minimization and/or avoidance of any gap in coverage area.

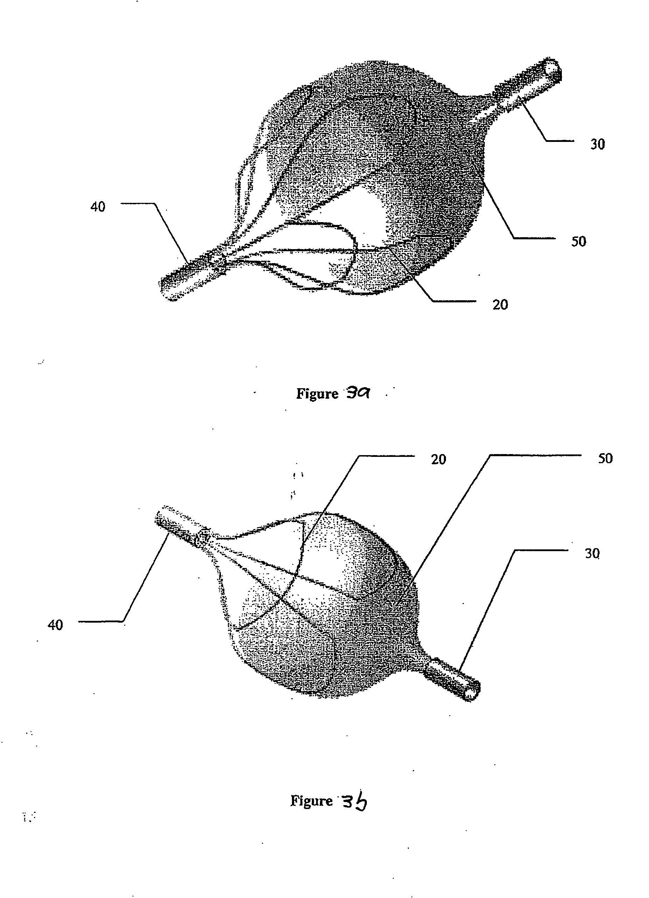

Figures 3a and 3b represent examples of two specific embodiments in accordance with the present invention with said supporting collar (30) present. Figure 3a is a four-loop configuration comprised of four independent strut loops (20). While figure 3b represents a three-loop configuration comprised of three strut loops (20). For each strut loop (20) the terminal starting and ending points of the loop (20) are fixed to the proximal support ring (40) while the distal circumferential portion of each respective loop is fixed to the proximal opening of the filter basket (50). In this exemplary embodiment, the distal support collar (30) is optional in the configurations shown. Furthermore, although not shown, filter membrane (50) can be optimized for filtering capacity by incorporating a combination of pores with consistent or varying sizes and distributions. In each exemplary embodiment, the device including both loops (20) and proximal support ring (40) may be cut from a single tube eliminating the need for separate structural components. As an example, laser cutting techniques for stent manufacturing can be employed to fabricate the embodiments described in accordance with the present invention. Cutting all or most of the structural components from a single tube by laser cutting or other appropriate methods provides significant cost savings as a result of the reduced number of manufacturing process steps. In certain instances, formed wire may also be used to fabricate the device in accordance with the present invention. Some device designs and shapes simply do not lend themselves to cost effective laser cutting and thus wire forming would be more cost effective. Supporting struts (20) can be fabricated from a number of biocompatible materials including metals, ceramics, and polymers. Preferable materials for the supporting struts (20) are shape memory metals and super-elastic alloys such as nitinol.

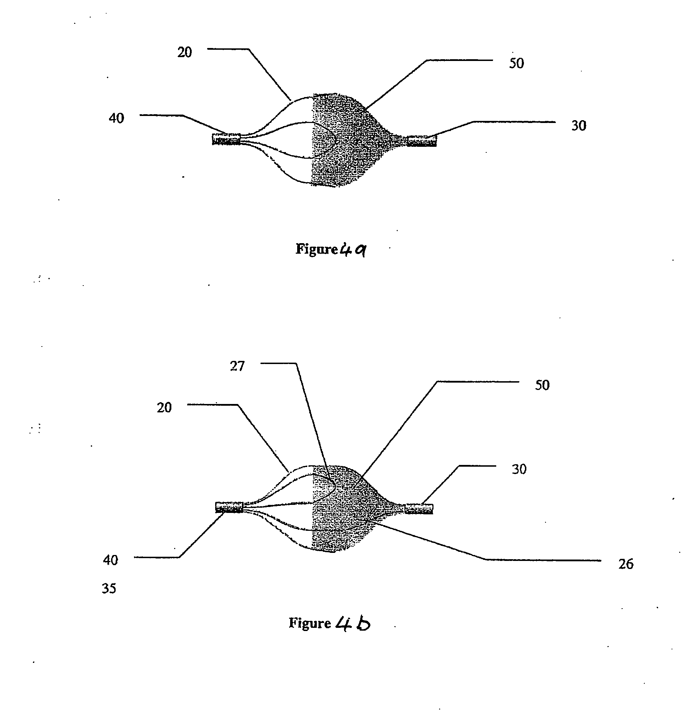

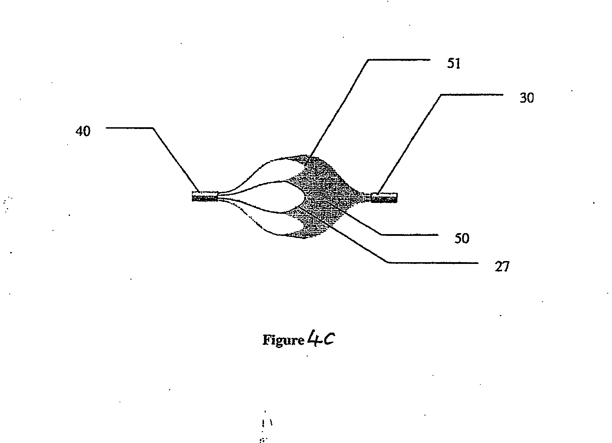

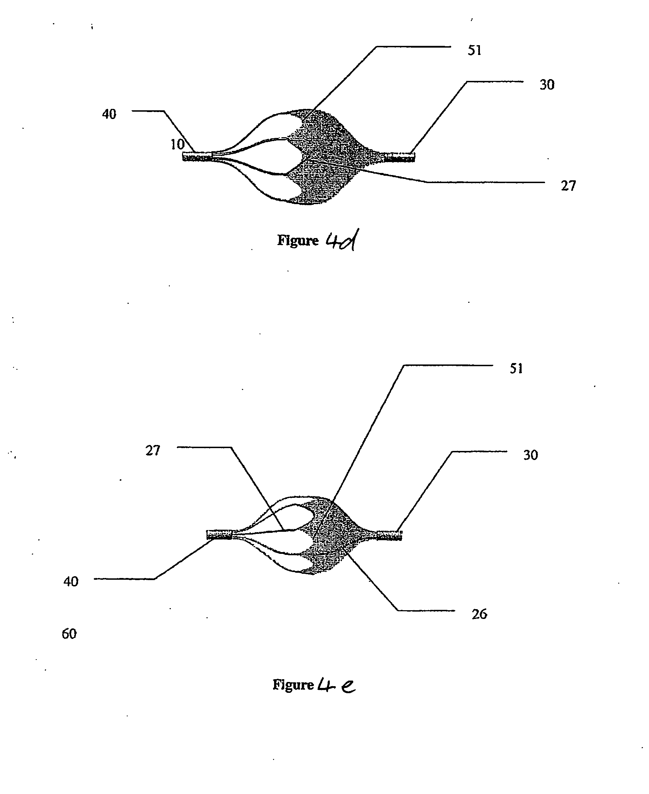

Additional embodiments are shown in figures 4a through 4e in which the three or four loop configuration can be augmented by providing additional filter support accomplished by strut member (26) as shown in figures 4b & 4e. These embodiments of the present invention are also capable of being fabricated from a single tube. Preferably these additional intermediate strut members (26) would be located between the struts having the loop configuration (27). Alternately, the strut loop configuration (27) can be a "U" type configuration as shown in figure 4c or a "V" type configuration as shown in figure 4d. Alternately, the membrane (50) can be scalloped (51) as shown in figures 4c, 4d & 4e, which can result in additional profile reductions without any decrease in filtering effectiveness.

## Claims

1. A vascular filtering device comprising:
a compliant substantially enclosed filter membrane (50) having a proximal opening;
a closure ring (40) having a concentric though hole adapted to slidably engage with a guide wire; and
two or more supporting struts (20) spaced apart from one another having proximal terminal ends, distal terminal ends, and substantially circumferential intermediate portions between the terminal ends, wherein the terminal ends are operatively attached to said closure ring (40);
**characterized in that** the circumferential intermediate portions of said supporting struts (20) are operatively attached to the proximal opening of said filter membrane (50) such that each supporting strut (20) can expand away from a guide wire independently of the or each other supporting strut (20) such that radial expansion of the filter membrane (50) is non-uniform.

2. The filtering device of claim 1 wherein the struts (20) are connected to the filter membrane (50) such that radial strut movement controls the radial expansion of the filter membrane (50) but allows for independent longitudinal movement of the struts (20) relative to the filter membrane (50).

3. The filtering device of claim 2 wherein the struts (20) are operatively attached to the filter membrane (50) via loops fixed to the filter membrane (50).

4. The filtering device of claim 1 wherein said membrane (50) is a polymeric film.

5. The filtering device of claim 4 wherein said polymeric film is polyurethane having at least one through hole.

6. The filtering device of claim 1 wherein said membrane (50) is a Nickel-Titanium Alloy thin film having at least one through hole.

7. The filtering device of claim 1 wherein the proximal opening of said membrane (50) is scalloped.

8. The filtering device of claim 1 further comprising:
a supporting collar (30) having a concentric though hole adapted to slidably engage with a guide wire; and
an axial supporting strut (26) having a proximal end and a distal end wherein the distal end of said axial supporting strut (26) is operatively attached to said supporting collar (30) and the proximal end of said axial supporting strut (26) is operatively attached to said closure ring (40).

9. The filtering device of claim 7 wherein the scalloped portion of the proximal opening of said membrane (50) substantially follows the circumferential portion of said supporting struts (20).

10. The filtering device of claim 7 wherein the scalloped portion of the proximal opening of said membrane (50) is located and extending between adjacent circumferential portions of said supporting struts (20).

11. The filtering device of claim 1 wherein said struts (20) are metal.

12. The filtering device of claim 1 wherein said membrane (50) incorporates an active compound.

13. The filtering device of claim 1 or 8 wherein said struts (20, 26) incorporate an active compound.

14. The filtering device of claim 1 wherein said struts (20) are polymeric.

15. The filtering device of claim 1 wherein said struts (20) are ceramic.

16. The filtering device of claim 11 wherein said metal is a shape-memory alloy.

17. The filtering device of claim 11 wherein said metal is a super-elastic alloy.

18. The filtering device of claim 16 wherein said shape-memory alloy is Nickel-Titanium Alloy.

19. The filtering device of claim 17 wherein said super-elastic alloy is Nickel-Titanium Alloy.

20. A method for fabricating a filtering device comprising the steps of:
providing elongated wire;
forming said elongated wire into strut members (20); and
attaching said strut members (20) to a closure ring (40) having a concentric through hole adapted to slidably engage a guide wire, wherein each strut member (20) has a proximal terminal end, a distal terminal end and a substantially circumferential intermediate portion between the terminal ends, wherein the terminal ends are operatively attached to said closure ring (40);
**characterized in that** the circumferential intermediate portions of said strut members (20) are operatively attached to a proximal opening of a compliant substantially enclosed filter membrane (50) such that each strut member (20) can expand away from a guide wire independently of the or each other strut member (20) such that radial expansion of the filter membrane (50) is non-uniform.

21. The method of claim 20 wherein said elongated wire is Nickel-Titanium Alloy.

## Patentansprüche

1. Vaskulärfiltervorrichtung, welche Folgendes umfasst:
eine nachgebende, im wesentlichen umschlossene Filtermembran (50) mit einer proximalen Öffnung;
einen Verschlussring (40) mit einem konzentrischen Durchgangsloch, welcher eingerichtet ist, um mit einem Führungsdraht verschiebbar einzukoppeln; und
zwei oder mehr Stützstreben (20), welche voneinander beabstandet sind und proximale Abschlussenden, distale Abschlussenden und im Wesentlichen periphere Zwischenabschnitte zwischen den Abschlussenden aufweisen, wobei die Abschlussenden wirksam an dem Verschlussring (40) angebracht sind;
**dadurch gekennzeichnet, dass** die peripheren Zwischenabschnitte der Stützstreben (20) wirksam an der proximalen Öffnung der Filtermembran (50) angebracht sind, so dass sich jede Stützstrebe (20) unabhängig von der oder jeder anderen Stützstrebe (20) von dem Führungsdraht weg ausdehnen kann, so dass eine radiale Ausdehnung der Filtermembran (50) ungleichförmig ist.

2. Filtervorrichtung nach Anspruch 1, wobei die Streben (20) mit der Filtermembran (50) verbunden sind, so dass eine radiale Strebenbewegung die radiale Ausdehnung der Filtermembran (50) steuert, aber eine unabhängige longitudinale Bewegung der Streben (20) relativ zu der Filtermembran (50) ermöglicht ist.

3. Filtervorrichtung nach Anspruch 2, wobei die Streben (20) über an der Filtermembran (50) befestigte Schlaufen wirksam an der Filtermembran (50) angebracht sind.

4. Filtervorrichtung nach Anspruch 1, wobei die Membran (50) ein Polymerfilm ist.

5. Filtervorrichtung nach Anspruch 4, wobei der Polymerfilm Polyurethan ist und zumindest ein Durchgangsloch aufweist.

6. Filtervorrichtung nach Anspruch 1, wobei die Membran (50) ein dünner Nickel-Titan-Legierungsfilm ist und zumindest ein Durchgangsloch aufweist.

7. Filtervorrichtung nach Anspruch 1, wobei die proximale Öffnung der Membran (50) einen Bogenrand aufweist.

8. Filtervorrichtung nach Anspruch 1, welche weiter Folgendes umfasst:
einen Stützkragen (30) mit einem konzentrischen Durchgangsloch, welcher eingerichtet ist, um verschiebbar mit einem Führungsdraht einzukoppeln; und
eine axiale Stützstrebe (26) mit einem proximalen Ende und einem distalen Ende, wobei das distale Ende der axialen Stützstrebe (26) wirksam an dem Stützkragen (30) angebracht ist und das proximale Ende der axialen Stützstrebe (26) wirksam an dem Verschlussring (40) angebracht ist.

9. Filtervorrichtung nach Anspruch 7, wobei der Abschnitt mit Bogenrand der proximalen Öffnung der Membran (50) im Wesentlichen dem peripheren Abschnitt der Stützstreben (20) folgt.

10. Filtervorrichtung nach Anspruch 7, wobei sich der Abschnitt mit Bogenrand der proximalen Öffnung der Membran (50) zwischen benachbarten peripheren Abschnitten der Stützstreben (20) befindet und sich dazwischen erstreckt.

11. Filtervorrichtung nach Anspruch 1, wobei die Streben (20) metallen sind.

12. Filtervorrichtung nach Anspruch 1, wobei die Membran (50) eine aktive Verbindung beinhaltet.

13. Filtervorrichtung nach Anspruch 1 oder 8, wobei die Streben (20, 26) eine aktive Verbindung beinhalten.

14. Filtervorrichtung nach Anspruch 1, wobei die Streben (20) polymer sind.

15. Filtervorrichtung nach Anspruch 1, wobei die Streben (20) keramisch sind.

16. Filtervorrichtung nach Anspruch 1, wobei das Metall eine Formgedächtnislegierung ist.

17. Filtervorrichtung nach Anspruch 1, wobei das Metall eine überelastische Legierung ist.

18. Filtervorrichtung nach Anspruch 16, wobei die Formgedächtnislegierung eine Nickel-Titan-Legierung ist.

19. Filtervorrichtung nach Anspruch 17, wobei die überelastische Legierung eine Nickel-Titan-Legierung ist.

20. Verfahren zum Herstellen einer Filtervorrichtung, welches die folgenden Schritte umfasst:
Bereitstellen eines länglichen Drahtes;
Bilden von Strebenelementen (20) aus dem länglichen Draht; und
Anbringen der Strebenelemente (20) an einen Verschlussring (40) mit einem konzentrischen Durchgangsloch, welcher eingerichtet ist, um einen Führungsdraht verschiebbar einzukoppeln, wobei jedes Strebenelement (20) ein proximales Abschlussende, ein distales Abschlussende und einen im Wesentlichen peripheren Zwischenabschnitt zwischen den Abschlussenden aufweist, wobei die Abschlussenden wirksam an dem Verschlussring (40) angebracht sind;
**dadurch gekennzeichnet, dass** die peripheren Zwischenabschnitte der Strebenelemente (20) wirksam an einer proximalen Öffnung einer nachgebenden im Wesentlichen umschlossenen Filtermembran (50) angebracht sind, so dass sich jedes Strebenelement (20) unabhängig von dem oder jedem anderen Strebenelement (20) von einem Führungsdraht weg ausdehnen kann, so dass eine radiale Ausdehnung der Filtermembran (50) ungleichförmig ist.

21. Verfahren nach Anspruch 20, wobei der längliche Draht eine Nickel-Titan-Legierung ist.

## Revendications

1. Dispositif de filtration vasculaire, comprenant :
une membrane filtrante compliante flexible sensiblement enfermée (50) possédant une ouverture proximale ;
une bague de fermeture (40) possédant un trou débouchant concentrique adapté pour entrer en prise de façon coulissante avec un fil guide ; et
deux, ou plus, supports (20) espacés les uns des autres possédant des extrémités terminales proximales, des extrémités terminales distales, et des parties intermédiaires sensiblement circonférentielles entre les extrémités terminales, dans lequel les extrémités terminales sont fixées de façon fonctionnelle à ladite bague de fermeture (40) ;
**caractérisé en ce que** les parties intermédiaires circonférentielles desdits supports (20) sont fixées de façon fonctionnelle à l'ouverture proximale de ladite membrane filtrante (50) de sorte que chaque support (20) puisse s'étendre pour s'éloigner d'un fil guide indépendamment de l'autre ou de chaque autre support (20) de sorte que l'extension radiale de la membrane filtrante (50) soit non uniforme.

2. Dispositif de filtration selon la revendication 1, dans lequel les supports (20) sont reliés à la membrane filtrante (50) de sorte que le mouvement de support radial contrôle l'extension radiale de la membrane filtrante (50) mais permette le mouvement longitudinal indépendant des supports (20) par rapport à la membrane filtrante (50).

3. Dispositif de filtration selon la revendication 2, dans lequel les supports (20) sont fixés de façon fonctionnelle à la membrane filtrante (50) par l'intermédiaire de boucles fixées à la membrane filtrante (50).

4. Dispositif de filtration selon la revendication 1, dans lequel ladite membrane (50) est un film polymère.

5. Dispositif de filtration selon la revendication 4, dans lequel ledit film polymère est du polyuréthanne possédant au moins un trou débouchant.

6. Dispositif de filtration selon la revendication 1, dans lequel ladite membrane (50) est un film mince d'alliage de nickel-titane possédant au moins un trou débouchant.

7. Dispositif de filtration selon la revendication 1, dans lequel l'ouverture proximale de ladite membrane (50) est festonnée.

8. Dispositif de filtration selon la revendication 1, comprenant en outre :
un collier de support (30) possédant un trou débouchant concentrique adapté pour entrer en prise de façon coulissante avec un fil guide ; et
un support axial (26) possédant une extrémité proximale et une extrémité distale dans lequel l'extrémité distale dudit support axial (26) est fixée de façon fonctionnelle audit collier de support (30) et l'extrémité proximale dudit support axial (26) est fixée de façon fonctionnelle à ladite bague de fermeture (40).

9. Dispositif de filtration selon la revendication 7, dans lequel la partie festonnée de l'ouverture proximale de ladite membrane (50) suit sensiblement la partie circonférentielle desdits supports (20).

10. Dispositif de filtration selon la revendication 7, dans lequel la partie festonnée de l'ouverture proximale de ladite membrane (50) est positionnée et s'étend entre des parties circonférentielles adjacentes desdits supports (20).

11. Dispositif de filtration selon la revendication 1, dans lequel lesdits supports (20) sont métalliques.

12. Dispositif de filtration selon la revendication 1, dans lequel ladite membrane (50) incorpore un composé actif.

13. Dispositif de filtration selon la revendication 1 ou 8, dans lequel lesdits supports (20, 26) incorporent un composé actif.

14. Dispositif de filtration selon la revendication 1, dans lequel lesdits supports (20) sont polymères.

15. Dispositif de filtration selon la revendication 1, dans lequel lesdits supports (20) sont céramiques.

16. Dispositif de filtration selon la revendication 11, dans lequel ledit métal est un alliage à mémoire de forme.

17. Dispositif de filtration selon la revendication 11, dans lequel ledit métal est un alliage super-élastique.

18. Dispositif de filtration selon la revendication 16, dans lequel ledit alliage à mémoire de forme est un alliage de nickel-titane.

19. Dispositif de filtration selon la revendication 17, dans lequel ledit alliage super-élastique est un alliage de nickel-titane.

20. Méthode pour fabriquer un dispositif de filtration, comprenant les étapes consistant à :
fournir un fil allongé ;
former ledit fil allongé en éléments de support (20) ; et
fixer lesdits éléments de support (20) à une bague de fermeture (40) possédant un trou débouchant concentrique adapté pour entrer en prise de façon coulissante avec un fil guide, dans laquelle chaque élément de support (20) possède une extrémité terminale proximale, une extrémité terminale distale et une partie intermédiaire sensiblement circonférentielle entre les extrémités terminales, dans laquelle les extrémités terminales sont fixées de façon fonctionnelle à ladite bague de fermeture (40) ;
**caractérisé en ce que** les parties intermédiaires circonférentielles desdits éléments de support (20) sont fixées de façon fonctionnelle à une ouverture proximale d'une membrane filtrante flexible sensiblement enfermée (50) de sorte que chaque élément de support (20) puisse s'étendre pour s'éloigner d'un fil guide indépendamment de l'autre ou de chaque autre élément de support (20) de sorte que l'extension radiale de la membrane filtrante (50) soit non uniforme.

21. Méthode selon la revendication 20, dans laquelle ledit fil allongé est un alliage de nickel-titane.
